# DEMANDE DE BREVET EUROPEEN

(11) **EP 2 581 085 A1**
(43) Date de publication de la demande: **17.04.2013**
(21) Numéro de dépôt: 12290334.7
(22) Date de dépôt: 09.10.2012
(51) Int. Cl.: A61K 31/5415, A61K 9/00, A61K 9/08, A61K 9/14, A61K 47/40

(54) **Composition pharmaceutique pour l'administration par voie nasale de la metopimazine**

(30) Priorité: 10.10.2011 FR 1103068
(71) Demandeur: Skiba, Mohamed, 76520 Montmain (FR)
(72) Inventeur: Skiba, Mohamed, 76520 Montmain (FR); Bounoure, Frédéric, 76000 Rouen (FR); Mallet, Éric, 76230 Bois-Guillaume (FR)

(57) **Abrégé**

La présente invention concerne une composition pharmaceutique pour l'administration par voie nasale de la métopimazine en solution ou sous forme de poudre.

## Description

La présente invention définit un nouveau moyen d'administration de la métopimazine par voie nasale notamment pour le traitement des nausées vomissements. Cette nouvelle voie d'administration est notamment intéressante dans le cas des gastro-entérites, du mal des transports ou de la chimiothérapie.

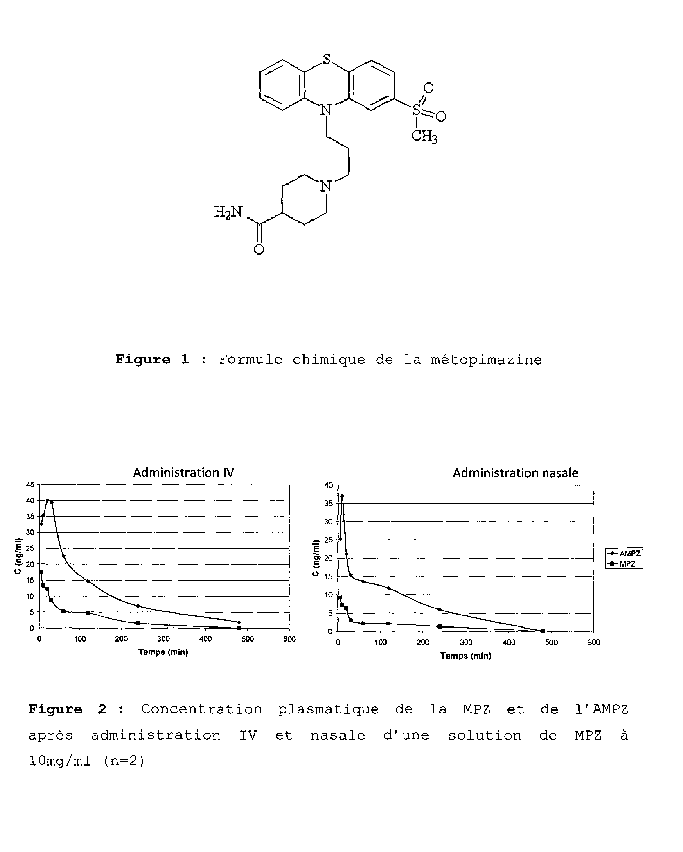

La métopimazine est un antiémétique appartenant à la classe chimique des phénothiazines, la métopimazine se caractérise par une activité anti-dopaminergique élective en raison de son passage très limité de la barrière hémato-encéphalique. Sa formule chimique est représentée dans la figure 1.

La métopimazine est un médicament antiémétique utilisé pour la prise en charge des nausées vomissements. De nombreuses formes pharmaceutiques sont commercialisées, ce qui peut laisser à penser que l'ensemble des besoins médicaux est satisfait. L'ensemble des formes pharmaceutiques disponibles est représenté dans le tableau 1

**Tableau n°1 : Différentes formes pharmaceutiques de métopimazine commercialisées en France**

| **Forme pharmaceutique** | **Indications** | **Type de patients** |
|---|---|---|
| Solution injectable 10mg/ml | Nausées et vomissements aigus chimio-induits | Adultes |
| Solution buvable 0.1% | Nausées et vomissements | Adultes |
| | | Enfants |
| | | Nourrissons |
| Gouttes buvables 0.4% | Nausées et vomissements | Adultes |
| | | Enfants |
| | | Nourrissons |
| Gélule 15mg | Nausées et vomissements | Adultes |
| Suppositoire 5mg | Nausées et vomissements | Adultes |
| | | Enfants |
| | | Nourrissons |
| Lyophilisat oral | Nausées et vomissements | Adultes |
| | | Enfants > 6 ans |

La solution de métopimazine injectable est utilisée dans la prévention et le traitement des nausées et vomissements induits par la chimiothérapie. Les autres formes sont notamment utilisées dans le traitement symptomatique des gastro-entérites ou du mal des transports. Hors dans un contexte de gastro-entérite se manifestant par une symptomatologie associant des vomissements et une diarrhée, une prise en charge par voie orale ou par voie rectale n'est pas possible. Ce besoin médical n'est que partiellement couvert par l'existence d'une forme à libération sublinguale contournant la voie orale et rectale. Cependant cette forme sublinguale ne peut être administrée aux enfants de moins de 6 ans. C'est pourquoi il est très intéressant de proposer une nouvelle forme pharmaceutique de métopimazine administrable par voie nasale notamment à l'enfant et au nouveau-né. Cette nouvelle forme de métopimazine présente une pharmacocinétique rapide proche de l'IV permettant un soulagement rapide du patient et une meilleure prise en charge. Elle est aussi pratique dans son utilisation notamment pour faciliter l'administration du médicament à l'enfant par les parents.

L'administration nasale des antiémétiques est connue depuis de nombreuses années. Le brevet US 5.760,086 a décrit le passage de nombreux antiémétiques avec la brompheniramine, la promethazine, la cyproheptadine, la dimenhydrinate, la meclizine, la cyclizine, la chlorcyclizine, la buclizine, la trimethobenzamide, la benzquinamide, le metoclopramide, la diphenhydramine, la doxylamine, la methapyrilene et la tripelennamine. Le brevet US 4,624,965 traite également de l'administration du metoclopramide par voie nasale dans le traitement des nausées vomissements chimioinduits. Cependant ce mode d'administration de la métopimazine n'a jamais été mis en évidence auparavant.

La composition pharmaceutique de la présente invention permet non seulement de remédier aux inconvénients connus de la forme injectable (IM), la forme rectal et de la forme orale mais également de proposer un service médical rendu supérieur permettant notamment l'amélioration de la qualité de vie des patients. La muqueuse nasale largement vascularisée est particulièrement adaptée à l'absorption rapide de la métopimazine pourvu que la frome pharmaceutique soit adaptée aux caractéristiques de ce principe actif.

Plus particulièrement, les compositions pharmaceutiques selon l'invention sont caractérisées en ce qu'elles contiennent la métopimazine ou l'un de ses sels pharmaceutiquement acceptables, éventuellement une cyclodextrine et l'un ou plusieurs excipients pharmaceutiquement acceptables.

Les compositions pharmaceutiques selon l'invention se présentent sous la forme de solution aqueuse ou de poudre administrables à l'homme à l'aide d'un dispositif approprie permettant de délivrer à chaque pulvérisation la quantité de la métopimazine nécessaire pour obtenir l'effet thérapeutique approprié.

Dans les compositions pharmaceutiques selon l'invention, la métopimazine se présente sous dorme base ou d'un sel pharmaceutiquement acceptable.

La métopimazine sera préférentiellement utilisé sous la forme sel.

Les cyclodextrines utilisables dans les compositions pharmaceutiques selon l'invention sont plus spécifiquement les α,β et γ-cyclodextrines, leurs dérivés ou des polymères de cyclodextrines, on peut citer à titre non limitatif les β-cyclodextrinesméthylées ou partiellement méthylées, l'hydroxypropoyl-β-cyclodextrines, ou la sulfobutyléther-β-cyclodextrines. Les cyclodextrines préférées sont les cyclodextrines partiellement méthylées est préférentiellement la cyclodextrines dont le degré de substitution par des groupements méthyles est voisin de 1,7 (RAMEB).

Dans les compositions pharmaceutiques en solutions selon l'invention, la quantité de la métopimazine (équivalent base) varie de 10 à 750 mg préférentiellement de 100 à 500 mg pour une solution aqueuse de 10 ml.

Les solutions aqueuses pourront être rendues isotoniques par addition de chlorure de sodium par exemple. Le pH des solutions aqueuses sera préférentiellement ajusté à 5,5 par addition d'un tampon citrate.

Dans les compositions pharmaceutiques en solutions selon l'invention, la quantité de la métopimazine (équivalent base) varie de 10 à 750 mg préférentiellement de 100 à 500 mg, la quantité de cyclodextrine varie de 70 à 3750 mg préférentiellement de 200 à 3000 mg pour une solution aqueuse de 5 ml.

Préférentiellement, pour une solution aqueuse finale de 5 ml, la quantité de la métopimazine (équivalent base) est égale à 100 mg et la quantité de cyclodextrine RAMEB est égale à 210 mg. Les solutions aqueuses pourront être rendues isotoniques par addition de chlorure de sodium par exemple. Le pH des solutions aqueuses sera préférentiellement ajusté à 5,5 par addition de tampon citrate

Les études précliniques réalisées chez le lapin avec des compositions pharmaceutiques selon l'invention ont montré une excellente tolérance et une meilleure biodisponibilité chez le lapin.

Les exemples suivants illustrent l'invention mais ne la limitent en aucune façon.

### EXEMPLE 1 :

### Formulation solution

Métopimazine : 10mg
NaCl : QSP 300mOsm
Tampon Citrate : QSP pH 5.5
Eau : QSP 1ml

Cette composition pharmaceutique est administrée au moyen d'une pompe doseuse délivrant 50 µl de solution soit 0,5 mg de métopimazine base à chaque pulvérisation.

### EXEMPLE 2 :

### Formulation solution

Métopimazine : 10mg
RAMEB : 21mg
NaCl : QSP 300mOsm
Tampon Citrate : QSP pH 5.5
Eau : QSP 1ml

Cette composition pharmaceutique est administrée au moyen d'une pompe doseuse délivrant 50 µl de solution soit 0,5 mg de métopimazine base à chaque pulvérisation.

### EXEMPLE 3 :

### Formulation poudre

Métopimazine micronisée : 10mg
Mannitol : 5mg

Cette composition pharmaceutique est administrée au moyen d'un spray-poudre délivrant 1,5 mg de poudre soit 1 mg de métopimazine base à chaque pulvérisation.

### EXEMPLE 4 :

### Formulation poudre

Métopimazine micronisée : 10mg
RAMEB : 5mg
Mannitol : 5 mg

Cette composition pharmaceutique est administrée au moyen d'un spray-poudre délivrant 2 mg de poudre soit 1 mg de métopimazine base à chaque pulvérisation.

### ETUDE PRECLINIQUE

Le passage par voie nasale de la métopimazine a été caractérisé chez l'animal dans un modèle de référence qu'est le lapin. Les lapins utilisés étaient des femelles de race néozélandaises provenant de chez Charles River (poids 3,5 kg). La formulation testée chez le lapin a été la suivante:
**Formule 1 :**
   Métopimazine : 10mg
   NaCl : QSP 300mOsm
   Tampon Citrate : QSP pH 5.5
   Eau : QSP 1ml

La posologie administrée au lapin a été calculée à partir de la dose pédiatrique soit 1 mg / kg / jour en 3 prises. Le poids des lapins étant de 3,5 kg, la dose par prise est d'environ 1 mg. La dose est donc administrée par instillation de 50 µl dans chaque narine d'une solution à 10 mg/ml.

Avant toute manipulation, le lapin est placé dans des cages de contention. Un cathéter est introduit sur chaque oreille du lapin au niveau de la veine marginale et d'une artère. Le cathéter veineux a permis d'injecter le soluté de remplissage (sérum physiologique) et le cathéter artériel a servi aux différents prélèvements sanguins tout au long de la manipulation. Les prélèvements ont été réalisés à temps réguliers : 0, 5, 10, 20, 30, 60, 120, 240 et 480 minutes. La préparation est administrée aux lapins en position supinale. Les lapins sont être maintenus dans cette position au moins 5 minutes après administration pour assurer le contact entre l'épithélium et la formulation. La solution nasale est administrée par instillation d'un volume de 50µL avec une micropipette dans chaque narine du lapin. Le volume du prélèvement est d'environ 3 mL. Après 2 h, les lapins sont placés dans leur cage avec un libre accès à la nourriture et à l'eau dans l'attente des prélèvements suivants. Le dosage est réalisé par CLHP avec détection par fluorescence en utilisant la méthode décrite par Angelo et al. J of Chrom, 496 ; 472-477, (1989). L'appareil de dosage est constitué d'une pompe Jasco, d'un injecteur Kontron et d'un détecteur de fluorescence Varian Prostar.

L'étude pharmacocinétique a été conduite sur le logiciel PK Solutions 2.

La formulation a été administrée par voie nasale. Les concentrations de métopimazine (MPZ) et de l'acide de métopimazine (AMPZ) sont représentées dans la figure 2. Le pic de métopimazine apparaît très rapidement avec un tmax à 5 minutes. La Cmax est moins importante que l'IV avec 9,2 ng/ml. La MPZ est rapidement métabolisée en AMPZ. Son Tmax est de 10 minutes avec une Cmax de 36,9 ng/ml.

La formule 1 a été comparée à la voie IV. La métopimazine a été administrée dans la veine marginale de l'oreille. Les concentrations plasmatiques de la MPZ et l'AMPZ sont représentées également dans la figure 2.

La métopimazine est détectée immédiatement avec un tmax observé de 5 minutes et une Cmax de 17,4 ng/ml. Le temps de demi-vie est de 72 minutes. Le métopimazine est rapidement desaminée en acide de métopimazine. Son tmax est de 20 minutes et sa Cmax de 40 ng/ml. Le temps de demi-vie est plus important avec 105 minutes.

Les paramètres pharmacocinétiques sont résumés dans le tableau 3

**Tableau 3 : Données de pharmacocinétiques de la MPZ et de l'AMPZ après administration nasale ou IV (n=2)**

| **AMPZ** | | | | |
|---|---|---|---|---|
| | t max (min) | C max (ng/ml) | AUC (ng/ml/min) | T1/2 (min) |
| IV | 20 | 40 | 5765 | 105 |
| Formule 1 | 10 | 36,9 | 3651 | 110 |

| **MPZ** | | | | |
|---|---|---|---|---|
| IV | 5 | 17,4 | 1403 | 72 |
| Formule 1 | 5 | 9,2 | 728 | 92 |

La biodisponibilité de la MPZ par voie nasale est de l'ordre de 50% pour la formulation testée. Cette biodisponibilité est plus importante que celle obtenue par voie rectale ou orale, respectivement 19,5% et 22,3%. Ces 2 voies présentent un effet de 1^{er} passage hépatique pouvant expliquer la différence de biodisponibilité.

### Exemple 2 :

Selon le même protocole, une autre formulation a été testée.
**Formule 2 :**
   Métopimazine : 10mg
   DMβCD : 21mg
   NaCl : QSP 300mOsm
   Tampon Citrate : QSP pH 5.5
   Eau : QSP 1ml

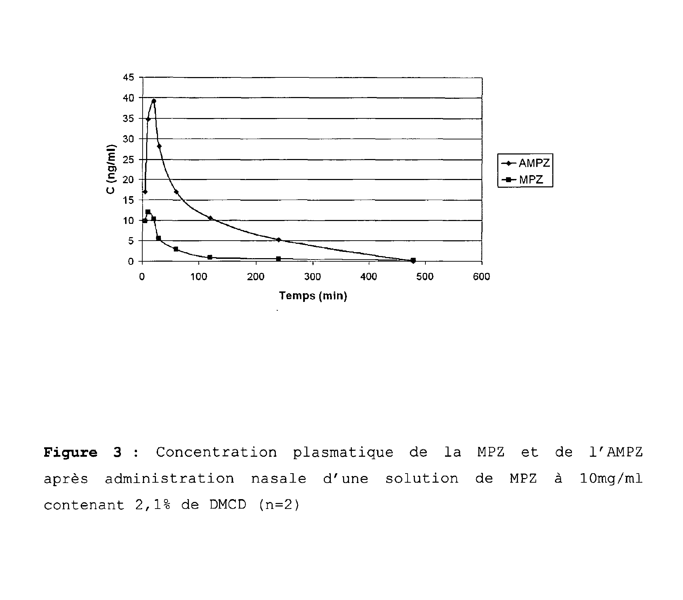

Les concentrations plasmatiques sont représentées figure 3. La voie nasale présente donc un profil pharmacocinétique proche de la voie IV. L'ensemble des paramètres pharmacocinétiques est repris dans le tableau 4.

**Tableau 4 : Données de pharmacocinétiques de la MPZ et de l'AMPZ après administration nasale ou IV (n=2)**

| **AMPZ** | | | | |
|---|---|---|---|---|
| | t max (min) | C max (ng/ml) | AUC (ng/ml/min) | T1/2 (min) |
| IV | 20 | 40 | 5765 | 105 |
| Formule 2 | 10 | 33,6 | 3972 | 82 |

| **MPZ** | | | | |
|---|---|---|---|---|
| IV | 5 | 17, 4 | 1403 | 72 |
| Formule 2 | 10 | 12,1 | 702 | 52 |

La biodisponibilité de la MPZ par voie nasale est de l'ordre de 50% pour cette nouvelle formulation. L'ajout de la RAMEB permet cependant d'augmenter la Cmax de la formule 2 par rapport à la formule 1.

### ETUDE DE LA TOLERANCE LOCALE

La métopimazine est déjà administrée par de nombreuses autres voies : orale, sublinguale, rectale, IV direct et IM sans problème de tolérance.

La tolérance a été étudiée chez le lapin après administration de 2mg de Formule 1 X3/j pendant 7 jours. Chaque lapin était son propre contrôle, puisqu'une narine recevait le Vogalène pendant que la seconde recevait une solution de sérum physiologique. Après l'exposition, les lapins sont sacrifiés afin de récupérer et d'étudier la pièce anatomique.

Les résultats ont été les suivants :

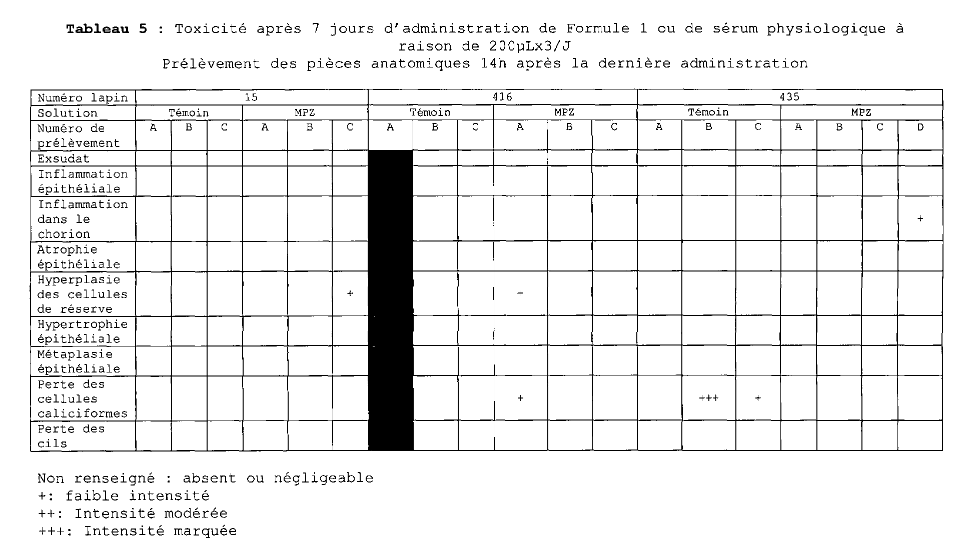

**Tableau 6 : Toxicité après 7 jours d'administration de Formule 1 ou de sérum physiologique à raison de 200µLx3/J Prélèvement des pièces anatomiques 70h après la dernière administration**

| Numéro lapin | 30 | | | | | | 52 | | | | | | 528 | | | | | |
|---|---|---|---|---|---|---|---|---|---|---|---|---|---|---|---|---|---|---|
| Solution | Témoin | | | MPZ | | | Témoin | | | MPZ | | | Témoin | | | MPZ | | |
| Numéro de prélèvement | A | B | C | A | B | C | A | B | C | A | B | C | A | B | C | A | B | C |
| Exsudat | | | | | | | | | | | | | | | | | | |
| Inflammation épithéliale | | | | | | | | | | + | | | | | | | + avec eosinophiles et lymphocytes | |
| Inflammation dans le chorion | | | | | | | | | | | | | | | | | | |
| Atrophie épithéliale | | | | | | | | | | | | | | | | | | |
| Hyperplasie des cellules de réserve | | | | | | + | + | | | + | + | | + | | | + | ++ | ++ |
| Hypertrophie épithéliale | | | | | | | | | | | | | | | | | | |
| Métaplasie épithéliale | | | | | | | | | | | | | | | | | | |
| Perte des cellules caliciformes | + | | | +++ | | ++ | | | ++ | +++ | | | +++ | + | | | + | + |
| Perte des cils | + | | ++ | ++ | | | | | | +++ | | | +++ | | | | + | + |

| | | | | | | | | | | | | | | | | | | |
|---|---|---|---|---|---|---|---|---|---|---|---|---|---|---|---|---|---|---|
| Non renseigné : absent ou négligeable +: faible intensité ++: Intensité modérée +++: Intensité marquée | | | | | | | | | | | | | | | | | | |

Les résultats sont en faveur d'une bonne tolérance locale puisqu'aucune différence significative n'est retrouvée entre la formule 1 et le placebo.

Cette étude a montré que la MPZ pouvait être administrée par voie nasale. Le profil pharmacocinétique est alors proche de celui de la voie IV avec une biodisponibilité de l'ordre de 50%. L'utilisation de cette voie augmente la quantité de MPZ desaminée en AMPZ.

## Revendications

1. Composition pharmaceutique sous forme de solution aqueuse, suspension ou poudre pour l'administration de la métopimazine **caractérisée en ce qu'**elle continent :
- de la métopimazine sous forme de base,
- éventuellement une cyclodextrine,
- un ou plusieurs excipients pharmaceutiquement acceptables.

2. Composition pharmaceutique selon la revendication 1 **caractérisée en ce que** la cyclodextrine est une b-cyclodextrine partiellement méthylée.

3. Composition pharmaceutique selon l'une quelconque des revendications 1 ou 2 **caractérisée en ce que** pour une solution aqueuse ou suspension finale de 10 ml, la quantité de la métopimazine est comprise entre 10 et 1500 mg.

4. Composition pharmaceutique selon l'une quelconque des revendications 1 à 2 **caractérisée en ce que** pour une solution aqueuse ou suspension finale de 10 ml, la quantité de la métopimazine est comprise entre 10 et 1500 mg pour quantité de cyclodextrine comprise entre 70 et 3700 mg.

5. Composition pharmaceutique selon l'une quelconque des revendications 1 ou 2 **caractérisée en ce que** lorsque cette composition est sous forme de poudre, la quantité de la métopimazine est comprise entre 0,1 mg et 50 mg.

6. Composition pharmaceutique selon l'une quelconque des revendications 1 ou 2 **caractérisée en ce que** lorsque cette composition est sous forme de poudre, la quantité de la métopimazine est comprise entre 0,1 et 50 mg pour une quantité de cyclodextrine comprise entre 5 et 70 mg.
